# EUROPEAN PATENT SPECIFICATION

(11) **EP 3 063 290 B1**
(45) Date of publication and mention of the grant of the patent: **21.11.2018**
(21) Application number: 14783547.4
(22) Date of filing: 09.10.2014
(51) Int. Cl.: C12Q 1/24, C12Q 1/04, C12Q 1/68

(54) **METHOD FOR ISOLATING MICROORGANISMS FROM A COMPLEX SAMPLE**
VERFAHREN ZUR ISOLIERUNG VON MIKROORGANISMEN AUS EINER KOMPLEXEN PROBE
PROCÉDÉ POUR ISOLER DES MICRO-ORGANISMES D'UN ÉCHANTILLON COMPLEXE

(30) Priority: 30.10.2013 EP 13290263
(43) Date of publication of application: 07.09.2016
(73) Proprietor: Merck Patent GmbH, 64293 Darmstadt (DE)
(72) Inventor: HOHNADEL, Marisa, 67800 Bischheim (FR); JOUETTE, Sebastien, 67100 Strasbourg (FR)
(86) International application number: PCT/EP2014/002736
(87) International publication number: WO 2015/062699

(56) References cited:
- EP-A2- 0 285 439
- WO-A1-2010/062349
- WO-A1-2012/168003
- LIQING ZHOU ET AL: "A novel method of selective removal of human DNA improves PCR sensitivity for detection of Salmonella Typhi in blood samples", BMC INFECTIOUS DISEASES, BIOMED CENTRAL, LONDON, GB, vol. 12, no. 1, 27 July 2012 (2012-07-27), page 164, XP021106912, ISSN: 1471-2334, DOI: 10.1186/1471-2334-12-164
- "Molecular Sepsis Diagnostics", Molzym , 2009, XP002732521, Retrieved from the Internet: URL:http://www.birdsrl.it/documents/brochu resepsis.pdf [retrieved on 2014-11-14]
- HANDSCHUR M ET AL: "Preanalytic removal of human DNA eliminates false signals in general 16S rDNA PCR monitoring of bacterial pathogens in blood", COMPARATIVE IMMUNOLOGY, MICROBIOLOGY AND INFECTIOUS DISEASES, PERGAMON PRESS, OXFORD, GB, vol. 32, no. 3, 1 May 2009 (2009-05-01), pages 207-219, XP025966511, ISSN: 0147-9571, DOI: 10.1016/J.CIMID.2007.10.005 [retrieved on 2008-02-07]
- FRIEDHOFF P ET AL: "A Procedure for Renaturation and Purification of the Extracellular Serratia marcescens Nuclease from Genetically Engineered Escherichia coli", PROTEIN EXPRESSION AND PURIFICATION, ACADEMIC PRESS, SAN DIEGO, CA, vol. 5, no. 1, 1 February 1994 (1994-02-01), pages 37-43, XP024799934, ISSN: 1046-5928, DOI: 10.1006/PREP.1994.1005 [retrieved on 1994-02-01]
- Eurogentec: "EurogEntEc HEadquartErs LIEGE SCIENCE PARK @BULLET 4102 Seraing @BULLET Belgium @BULLET", , 6 October 2009 (2009-10-06), XP055437014, Retrieved from the Internet: URL:https://secure.eurogentec.com/EGT/file s/TDS ME0280-.pdf [retrieved on 2017-12-20]

## Description

The present invention relates to a method for isolating and optionally detecting microorganisms from a sample comprising mammalian cells, comprising the use of a lysis buffer comprising Benzonase®Nuclease. The invention further relates to the use of a lysis buffer comprising Benzonase®Nuclease.

Microbial bioburden monitoring and timely results are critical for the quality control during the production of recombinant proteins or other biotechnological products coming from a bioreactor.
Typically the bioburden level of a bioreactor sample is determined by membrane filtration and subsequent incubation on culture media or poor plating. Due to the particulate content of a bioreactor sample, especially when a high level of mammalian cells is present in the bioreactor, only small amounts of sample can be filtered (1ml to 2ml or even less), requiring multiple filtration steps in order to be able to test a representative volume of each sample.
This method can be improved by adding a differential centrifugation step for separating mammalian cells and microorganisms. In this case, the supernatant containing the microorganisms is filtered on a membrane and incubated on culture media. The drawback of this method is the possible loss of some contaminants which may be trapped with or sticked to the mammalian cells.
For diagnostic purposes, high sample volumes are needed in order to be able to detect low level contamination. The unsatisfactory membrane filterability resulted in the development of lysis buffers capable of differentially lysing the eukaryotic cells of a clinical sample without lysing contained microorganisms. After lysis buffer treatment, the sample is filtered and the membrane is incubated on culture media. In some cases, molecular methods are used for faster detection of microorganisms. In this case, a specific treatment needs to be applied after the lysis of the eukaryotic cells to remove high contents of eukaryotic DNA which might interfere with the detection method. In a following step the microorganisms are lysed to release their DNA. This DNA is purified and then detected by molecular methods such as PCR or real-time PCR.The combination of all these steps is time consuming and laborious due to many manual handling steps.
In the state of the art solutions for isolating and detecting microbial contamination of mammalian cell samples by selectively lysing mammalian cells are described. These publications relate to the detection of microbial contaminations in clinical samples, such as body fluids, in particular blood.
For example, WO 2009/015484 A1 discloses a rapid method for isolating and detecting microorganisms and microorganisms nucleic acids from blood by selectively lysing the blood cells while protecting microbial cells using a saponin formulation.
In WO 2011/070507 A1 the selective lysis of blood cells in a sample is effectuated by incubating the sample in a non-ionic detergent under alkaline conditions.
US 2008/0131955 A1 describes a method for separating target DNA from mixed DNA in a body fluid sample involving a step of selectively lysing mammalian cells.
EP 0745849 A2 discloses a method for processing whole blood for nucleic acid analysis of microorganisms which may be present in the sample, wherein red blood cells are selectively lysed.
Zhou et al. (BMC Infectious Diseases 2012, vol. 12, no. 1, page 164) is directed to a method for detection of Salmonella Typhi in blood samples.
The product brochure from Molzym GmbH & CO. KG (http://www.birdsrl.it/documents/brochuresepsis.pdf) shows a kit (MolYsis) for treating blood by removing human DNA and enriching bacterial DNA. Handschur et al. (Comparative Immunology, Microbiology and Infectious Diseases, 2009, 32, 207-219) discloses the removal of human DNA in order to detect bacterial pathogens in blood.

WO 2012/168003 A1 describes the selective lysis of eukaryotic cells in a sample comprising micro-organisms. The sample is a mammalian blood sample.
WO 2010/062349 A1 discloses a method for separation and characterization of microorganisms in a sample including a step of lysing non-microorganism cells. The method described herein focuses especially on blood-containing or clinical samples. Furthermore, non-clinical samples such as food, beverage, cosmetics etc. are mentioned.
EP0285439 A2 describes the separation of bacteria from white blood cells. The product brochure from Eurogentec referring to Benzonase®Nuclease (http://secure.eurogentec.com/EGT/files/TDS ME0280-.pdf) describes the different operating conditions for the enzyme.

However, complex bioreactor samples of large volumes and high cell density pose still a challenge with regard to the detection of microbial contaminations. The object of the present invention was therefore the development of a method for isolating and/ or detecting microbial contamination in complex samples comprising mammalian cells.

In a first aspect, the present invention relates to a method for isolating microorganisms from a sample comprising mammalian cells, comprising
a) contacting the sample with a lysis buffer comprising Benzonase®Nuclease,
b) obtaining the microorganisms by removing the mammalian cells, as defined in claim 1.

In a second aspect, the present invention relates to a method for isolating and detecting microorganisms from a sample comprising mammalian cells, comprising
a) contacting the sample with a lysis buffer comprising Benzonase®Nuclease,
b) obtaining the microorganisms by removing the mammalian cells,
c) performing a universal lysis on the microorganisms,
d) performing a specific or universal detection method for detecting the microorganisms,
as defined in claim 2.

The methods according to the present inventions provide several advantages:
- Quick analysis of large sample volumes (up to 50 ml; isolation of bacteria in less than 20 min),
- Detection of microorganisms with a good sensitivity in samples of high quantities of mammalian cells (up to 5*10⁸ cells total),
- simple and limited manual operations.

The sample comprising mammalian cells may be any clinical or non-clinical sample in which microorganism presence and/or growth is or may be suspected, as well as samples of materials that are routinely or occasionally tested for the presence of microorganisms. The sample may be a fresh or a frozen sample. According to the present invention the sample is a bioreactor sample.
Any kind of bioreactor can be considered in view of the present invention. The sample can be obtained from the bioreactor by any method known by the person skilled in the art, e.g. by transferring a smaller amount of the cell culture into a vessel. The vessel may be any tube or reaction vessel suitable for the method.

The quantity of the mammalian cells in the sample is at least 10⁶ cells. In a preferred embodiment the quantity of the mammalian cells in the sample is at least 10⁷ cells.
A typical range for the quantity of total mammalian cells in the sample is from 10⁶ cells to 5*10⁸ cells, e.g. around 10⁷ cells, 5*10⁷ cells, or 10⁸ cells.

The amount of sample utilized may vary greatly due to the versatility and sensitivity of the method. Appropriate volumes will depend on the source of the sample and the anticipated level of microorganisms in the sample. The method according to the present invention is suitable for smaller as well as for bigger sample volumina.
In a preferred embodiment of the present invention the volume of the sample to be analysed is at least 5 ml. More preferably, the volume of the sample is in a range of 5-50 ml, preferably 5-30 ml. An advantage of the present invention is the fact that even large volume samples can be analysed with high sensitivity. In a further embodiment of the present invention the volume sample is at least 10 ml or at least 20 ml.

In a first step of the method according to the present invention the sample is contacted with a lysis buffer comprising Benzonase®Nuclease.

Benzonase®Nuclease is a genetically engineered endonuclease from *Serratia marcescens*, degrading non-specificly all forms of DNA and RNA. The enzyme consists of two subunits of 30 kDa each. Benzonase®Nuclease is commercially available from Merck Millipore (article number 70664-3, 71206-3).

The lysis buffer efficiency is related to the cell quantity of the sample. The lysis buffer therefore comprises Benzonase®Nuclease in a concentration of 2.5 U to 175 U per sample (referring to the final concentration after addition to the sample). Preferably, the concentration is 2.5 - 100 U, more preferably 2.5 - 50 U and most preferably 10 - 30 U. The very preferred concentration of Benzonase®Nuclease is around 22.5 U. One U (unit) of Benzonase®Nuclease is defined as the amount of enzyme that results in a ΔA260 of 1.0 in 30 min, which corresponds to complete digestion of 37 µg DNA.

The necessary ingredients of the lysis buffer used for cell lysing depend on the type of mammalian cells to be lysed in the sample. Besides Benzonase®Nuclease, further ingredients of the lysis buffer are, e.g., PBS (phosphate buffered saline; PBS 1X: 137 mM NaCl, 10mM Phosphate, 2.7 ml KCl, pH 7.4), SDS (sodium dodecyl sulfate), urea, MgCl₂.

SDS can be replaced by similar substances, e.g. lithium dodecyl sulfate.

In a preferred embodiment of the present invention the buffer does not contain tris HCl, EDTA, EGTA, triton X and/or NP 40. Such buffer is advantageous since the the destruction of bacteria, yeast and molds is avoided.

According to the present invention the lysis buffer comprises the following ingredients, wherein all concentrations refer to the final concentration after addition to the sample:
- 0.05 to 0.5 % by weight SDS, preferably 0.5 %,
- 0.5 M to 4 M urea, preferably 0.5 M,
- 0.5 mM to 10 mM MgCl₂, preferably 2mM,
- 2.5 to 175 U Benzonase®Nuclease, preferably 22.5 U,
- PBS 1X - PBS 1/8 X, preferably 1/8 X.

The preparation of the lysis buffer is common to a person skilled in the art. Typically, the ingredients are dissolved and mixed. The buffer without the enzyme is typically filtered and/or heated prior to its use. The enzyme is then added in a second step.

The mammalian cells of the sample are typically cells growing in suspension culture, i.e. without being attached to a surface. Such cells may be cells naturally living in suspension, e.g. blood cells, or cells that have been modified to be able to survive in suspension cultures in high densities. Alternatively, cell lines growing on microcarriers can be used.

The term "mammalian cell" as used herein refers to any cell derived from a mammalian subject, including, but not limited to, humans, non-human primates, cattle, sheep, goats, pigs, horses, cats, dogs, rabbits, rodents (e.g. rats or mice), etc.
Examples of mammalian cell lines that can be used according to the present invention are hybridoma cell lines : e.g CRL-8018, CF-10H5 and many other cell types : e.g CHO (Chinese Hamster Ovary) cells, NSO cells (Murine myeloma cell line), Sp2/0 cells (Murine myeloma cell line), HT-1080 cells (fibrosarcoma cell line), MDCK (Madin-Darby canine kidney), HeLa (human epithelial cells) or Vero (kidney epithelial cells).

The sample to be analysed comprises or is suspected to comprise microorganisms. The microorganisms can be present in the bioreactor medium and/or endogenously in the mammalian cells (host cells). The microorganisms being isolated and/or detected according to the method of the present invention may be bacteria, yeast, fungi and/or combinations thereof. The microorganisms may be aerobic and/or anaerobic. Microorganisms of the present invention include, but are not limited to, the *Acinetobacter* genus, *Bacteroides* genus, *Burkholderia* genus, *Capnocytophaga* genus, *Clostridium* genus, *Corynebacterium* genus, *Citrobacter* genus, *Enterobacter* genus, *Enterococcus* genus, *Escherichia* genus, *Haemophilus* genus, *Klebsiella* genus, *Proteus* genus, *Pseudomonas* genus, *Salmonella* genus, *Serratia* genus, *Staphylococcus* genus, *Stenotrophomona* genus, *Streptococcus* genus, *Saccharomyces* genus, *Zygosaccharomyces* genus, *Penicillium* genus, *Aspergillus* genus and/or *Candida* genus.
Exemplary microorganisms may be *Escherichia coli, Enterococcus faecalis, Salmonella enterica, Staphylococcus aureus, Staphylococcus epidermidis, Pseudomonas aerugionosa.* Examples of yeast are *Candida albicans, Saccharomyces cerevisiae, Zygosaccharomyces bailii, Dekkera anomala.* Examples of fungi are *Aspergillus brasiliensis, Penicillium chrysogenum.*

In step a) the sample is contacted with the lysis buffer comprising Benzonase®Nuclease. The volume of the lysis buffer can be added to the sample in one or several steps. Similarly, it is possible to add the ingredients of the lysis buffer in one or several steps to the sample. For example, Benzonase®Nuclease may be added in a first step, followed by the addition of the remaining lysis buffer ingredients in a second step. Typically, after contacting the sample with the lysis buffer the sample is homogenized or mixed, e.g. by inverting the vessel several times and/or vortexing the vessel. In addition, a incubation step without agitation can be done, e.g. for 2 to 10 min, preferably around 5 min.

In step b) of the method according to the present invention the microorganisms are obtained by removing the mammalian cells. This step may involve several washing and concentrating steps. Concentration methods may for example include centrifugation, filtration, surface binding and/or magnetic trapping.
In a preferred embodiment, for collecting the microorganisms, the mammalian cells are removed by centrifugation, e.g. at 10000-20000 g, preferably 15000 g, for 5 to 15 min, preferably around 8 min. In a following step the supernatant is removed, e.g. by using a pipette or by a decanting step. The remaining pellet comprises the microorganisms.

Optionally, a washing step may follow depending on the type of sample. Typically, a suitable washing buffer (e.g. Tris) is added in a suitable amount (e.g. 5-20 ml). The sample is then centrifuged and the supernatant is removed from the pellet.

According to the present invention the microorganisms isolated after step b) may optionally be detected in two additional steps c) and d):
In step c) a universal lysis is performed on the microorganisms in order to extract and/or purify their nucleic acids. Various methods known in the art may be employed to extract DNA, e.g. the methods disclosed in Sambrook et al. (1989) Molecular cloning: a laboratory manual, 2nd ed. Cold Spring Harbor Laboratory Press, Cold Spring Harbor, N.Y. Cell lysis may be accomplished by standard methods, e.g. by enzymatic methods, bead methods, sonication, detergent methods or combinations thereof.

Suitable enzymes for enzymatic cell lysis are, e.g., lysozyme, lysostaphin, zymolase, mutanolysin, glycanases, proteases.

Beads suitable for cell disruption are of glass, ceramic, zirconium, or steel. After the addition of beads to the cells agitation by stirring or shaking of the mix is applied. Agitation can for example be applied by a common laboratory vortex mixer or in a specially designed clamp.

Detergent-based cell lysis results in the disruption of the lipid barrier surrounding cells. Suitable detergents may be chosen from non-ionic, zwitterionic and ionic detergents, e.g. CHAPS, Triton® X or TWEEN®. Preferably, ionic detergents are used. An example for a suitable ionic detergents is SDS.

In addition to the choice of detergent, other important considerations for optimal cell lysis include the buffer, pH, ionic strength and temperature: The lysis solution typically has a pH value greater than 5 and lower than 9, preferably greater than 6 and lower than 8, more preferably between 6.5 and 7.5.
The buffer which may be used is preferably selected from the group of phosphate buffer, phosphate buffered saline buffer (PBS), 2-amino-2-hydroxymethyl-1, 3-propanediol (TRIS) buffer, TRIS buffered saline buffer (TBS) and TRIS/EDTA (TE).

Optionally, one or more chelating agents can be added to the lysis solution to sequester divalent cations. Suitable chelators are, e.g., EDTA (ethylenediamine tetraacetic acid), EGTA (ethylene glycol tetraacetic acid) or ethylenediamine. Preferably, EDTA is used.

In a preferred embodiment of the present invention lysis is done mechanically, particularly preferred by sonication. Before sonication, the pellet is typically resuspended and/or washed with suitable buffer solutions as described above.

Sonication is generally effectuated in a sonicator, involving ultrasound application (typically 20-50 kHz) onto the sample. Any sonicator suitable for cell lysis can be used according to the present invention, including sonicators with probes coming into direct contact with the sample and sonicators applying the force externally to the sample. Preferably, sonicators with external application are used in order to avoid sample contamination. As an example, a sonicator as disclosed in unpublished EP 13290200.8 can be used. Further sonicators which can be used according to the present invention are commercially available, e.g. VialTweeter (Hielscher) or Bioruptor®Pico.
Typical parameters for sonication can easily be selected by a person skilled in the art.

Depending on the lysis method, a centrifugation step may follow: If the lysis is performed mechanically, e.g. via sonication, centrifugation is necessary in order to collect the lysate at the bottom of the tube. If beads are used for lysis, a centrifugation step is also needed in order to collect the beads at the bottom of the tube and to separate them from the lysate. A skilled person can easily determine the parameters for centrifugation. Typically, centrifugation is carried out as dislosed in Sambrook et al. (1989) Molecular cloning: a laboratory manual, 2nd ed. Cold Spring Harbor Laboratory Press, Cold Spring Harbor, N.Y.
No centrifugation step is needed if lysis is performed chemically, e.g. by using an enzyme.

After cell lysis the nucleic acids (DNA, RNA) may be precipitated by adding an alcohol, preferably ethanol or isopropanol. Additionally, the DNA or RNA can be provided in a form which is suitable for amplification using a commercially available DNA or RNA isolation kit, such as the NucleoSpin® tissue kit and the support protocol for Gram-positive bacteria (Machery-Nagel, Düren, Germany), the Nexttec® kit for genomic DNA from bacteria (Nexttec GmbH Biotechnologie, Leverkusen, Germany), magnetic beads for DNA purification (MilliPrep®kit by Merck Millipore), the QIAamp DNA mini kit (Qiagen) or the RNeasy Protect Bacteria Mini kit (Qiagen).

In step d) a specific or universal detection method is performed for detecting the microorganisms. The detection method may comprise, e.g., amplifying the microorganisms' nucleic acid and detecting the nucleic acid. The amplification of the nucleic acid sequences may be accomplished by any in vitro nucleic acid amplification technique known in the art. Such techniques may include methods requiring temperature cycling (such as PCR, real-time PCR, reverse transcription PCR, ligase chain reaction, transcription based amplification), and/or isothermal amplification systems.

In a preferred embodiment of the present invention amplification of the microorganisms' nucleic acid is performed by polymerase chain reaction (PCR) and/or any variants thereof.
Preferably, real-time PCR (rtPCR or qPCR) is applied. Real-time PCR is a method for detecting and measuring products generated during each cycle of a PCR, which are proportionate to the amount of template nucleic acid prior to the start of PCR. The information obtained, such as an amplification curve, can be used to quantify the initial amounts of template nucleic acid sequence.
The detection is preferably based on monitoring fluorescence at every cycle at a set temperature. Fluorescence is usually monitored using an optical device to collect the data at specific excitation and emission wavelengths for the particular fluorescent dye present in the sample. The cycle at which the fluorescence from a sample crosses the threshold for detection of fluorescence above background is called the cycle threshold, Ct, and allows the quantification of the starting template.

The PCR conditions are not particularly restricted but optimal conditions may be selected for each PCR apparatus. For example, the following conditions may be used:
- Thermal denaturation of double-stranded DNA to single-stranded DNA: Heating is generally made at about 90-98°C, preferably at about 92-96°C, generally for about 3 seconds to 1 minute, preferably for about 30 seconds to 1 minute.
- Annealing: Heating is made generally at about 40-70°C, preferably at 55-65°C, generally for about 5 seconds to 2 minutes, preferably for about 30 seconds to 90 seconds.
- DNA elongation reaction: Heating is made generally at about 60-75°C, preferably at about 70-74°C, generally for about 10 seconds to 3 minutes, preferably for about 30 seconds to 2 minutes.
- Mg ion concentration in the reaction liquid: Generally about 1-5 mM, preferably about 1.5-3.5 mM.

This reaction is typically carried out in about 20-50 cycles, preferably in about 45 cycles, whereby the target DNA can be amplified to a detectable level.

In the case of the real-time reverse-transcription PCR, an additional step is performed upfront the classical PCR conditions in order to transcribe the RNA into DNA thanks to a reverse transcriptase enzyme thus making PCR analysis of RNA molecules possible.

Any commercial PCR apparatus can be used. Amplification may be performed using primers and/or a collection of primers that may be selected from those capable of specific binding to nucleic acids of at least one microorganisms. In general, the term "primer" refers to a short nucleic acid molecule, such as a DNA oligonucleotide of 9 nucleotides or more in length, that is complementary to a section along a strand of the target nucleic acid, wherein the purpose of the primer is to initiate the nucleic acid replication of a longer nucleic acid along the strand. Suitable primers can easily be identified by a person skilled in the art.

The amplified nucleic acid of the microorganisms may be detected by hybridizing a probe and/or a collection of probes capable of specific binding (hybridizing) to the amplified nucleic acids.
According to the present invention the term "hybridize" means to detectably and specifically bind. Polynucleotides, oligonucleotides and fragments thereof selectively hybridize to nucleic acid strands under hybridization conditions that minimize appreciable amounts of detectable binding to nonspecific nucleic acids.
The fluorescent labelled probes typically used for real time PCR are e.g. LightCycler (hybridisation) probes, molecular beacons or hydrolysis probes (also called TaqMan® probes).
LightCycler probes utilize the technique of fluorescence resonance energy transfer (FRET). For this method two different oligonucleotide probes bound to a FRET donor and a FRET acceptor, respectively, are used for each target sequence. These probes bind side by side to the target sequence, bringing the fluorophors in proximity.
Molecular beacons (Tyagi et al., Nat. Biotechnol. 14:303-8, 1996) are oligonucleotide probes bound to a reporter fluorophor and a quencher. The nucleotides on the 5' end are complementary to the nucleotides on the 3' end, forming a stem loop structure. Because of the proximity of the fluorophor and the quencher no fluorescence is observed. Hybridization of the probe with the target sequence during real-time PCR leads to an increase in the reporter-quencher distance resulting in fluorescence of the reporter.

Preferably, hydrolysis probes (TaqMan® probes) are used (Lee et al., Nucleic Acids Res. 21:3761-6, 1993). These probes utilize as well the technique of fluorescence resonance energy transfer (FRET). The probes exhibit a fluorescent reporter at one end and a quencher of fluorescence at the opposite end. Because of the close proximity of the reporter to the quencher detection of the reporter fluorescence is suppressed. During the annealing stage of the PCR both primers and the probe anneal to the DNA target. Polymerization of a new DNA strand leads to the degradation of the probe by the 5'-3' exonuclease activity of the polymerase and physical separation of the fluorescent reporter from the quencher, resulting in an increase in fluorescence. Fluorescence can be detected and measured in the real-time PCR thermocycler, and its geometric increase corresponding to exponential increase of the product is used to determine the threshold cycle (Ct) in each reaction (see below).
Exemplary reporters include, but are not limited to: 6-carboxyfluorescein; carboxyfluorescein (FAM); boron dipyrromethene difluoride (BODIPY); acridine, stilbene, 6-carboxy-fluorescein (HEX), TET (Tetramethyl fluorescein), 6-carboxy-X-rhodamine (ROX), Rhodamine-6G, Texas Red, 2',7'-dimethoxy-4',5'-dichloro-6-carboxyfluorescein (JOE), Cy®3, Cy®5, VIC® (Applied Biosystems), LC Red 640, LC Red 705, Texas Red, Yakima Yellow®, as well as derivatives thereof.
Exemplary quenchers include, but are not limited to Black Hole Quenchers (WO 01/86001 A1) as BHQ1™ and BHQ2™, MGB (Minor-groove-binder, EP 0819133 B1), N,N,N',N'-tetramethyl-6-carboxyrhodamine (TAMRA), Eclipse® Dark Quencher, DABCYL, DABSYL, DDQ I and DDQ II.
A person skilled in the art can easily chose a suitable reporter-quencher combination. Typically, the absorption spectrum of the quencher needs to have a good overlap with the emission spectrum of the reporter in order to allow for optimal quenching.

The fluorescent signals generated may then be qualitatively and / or quantitatively analysed. The detection is preferably based on monitoring fluorescence at every cycle at a set temperature. Fluorescence is usually monitored using an optical device to collect the data at specific excitation and emission wavelengths for the particular fluorophors present in the sample.

The present invention provides high sensitivity for the isolation and optionally detection of microorganisms in a sample of mammalian cells. A particular advantage of this method is that the sensitivity of detection is even given when samples of very high quantities of mammalian cells are to be analyzed (up to 5*10⁸ cells in total in samples of up to 50 ml) and/or the samples comprise any few microorganism contamination. The method is very quick and simple: the microorganisms can be isolated in less than 20 min with only few manual operations. The entire sample preparation can be done in less than 1 hour. The method according to the present invention works on fresh as well as on frozen samples without affecting the qPCR sensitivity, whereas many methods according to the state of the art exhibit a poor performance on frozen samples.

According to the present invention, the ingredients of the lysis buffer are adjusted so that their final concentration after addition of the lysis buffer to the sample is:
- 0.05 to 0.5 % by weight SDS
- 0.5 M to 4 M urea
- 0.5 mM to 10 mM MgCl₂
- 2.5 to 175 U Benzonase®Nuclease
- PBS 1X - PBS X1/8.

In a more preferred embodiment the final concentration of the ingredients is:
- 0.5 % SDS,
- 0.5 M urea,
- 2 mM MgCl₂,
- 22.5 U Benzonase®Nuclease,
- PBS 1/8 X.

A premixed lysis buffer may comprise the following ingredients (concentration referring to the premixed lysis buffer before addition to the sample):
- 4 % SDS,
- 4 M urea,
- 16 mM MgCl₂,
- PBS 1X.
Benzonase®Nuclease may be added separately to the sample in a suitable amount to obtain the final use concentration of preferably 22.5 U.

In a further aspect, the present invention relates to the use of a lysis buffer as described above in a method as decribed above.

The following examples describe practical applications of the invention.

### Examples:

### Example 1: Matrix lysis protocol

Different volumes of mammalian cell samples are tested by the following protocol:

### Isolation of microorganism cells:

X ml (see table 1) of CHO cell samples are filled into a 50ml Corning tube. The sample is contaminated with microorganisms and homogenized by inverting the tube several times.

22.5U Benzonase®Nuclease (available from Merck Millipore, article number 70664-3 or 71206-3) is added (this enzyme should be diluted with nuclease free water in order to add a sufficient volume) and the sample is homogenized by inverting the 50ml tube several times.
The tube is filled with Y ml of buffer (4M urea, 4% SDS, 16mM MgCI2, x1 PBS) to obtain in the sample the final concentration of the buffer: 0.5M urea, 0.5% SDS, 2mM MgCI2, 1/8 PBS (see table 1). The sample is homogenized by inverting the tube several times, followed by an incubation step during 5min at room temperature without agitation. If there are any particles in suspension, a quick vortex step in order to dissolve the particles is applied. The sample is centrifuged for 8min at 15000g. The supernatant is removed with a pipette.
10ml 10mM Tris pH 7 are added and the sample is vortexed.

### Detection of microorganism cells:

The sample is centrifuged for 6min at 15000g. The supernatant is removed. 500µl of buffer solution (1M Guanidine HCI, 5mM Tris pH8 (+0.5 mM EDTA), 10mM EDTA, 0.5 % NLS (N-Lauroylsarcosine sodium salt solution)) are added and the sample is vortexed.
The tube is sonicated for 3min.
The tube is centrifuged during 1min at 3000g (optional) and is transferred into the nucleic acid purification instrument (Kingfisher™, ThermoScientific). The DNA is further purified with the MilliPrep magnetic bead kit for Mycoplasma (Merck-Millipore, article number MPRPBD048) (elution into 50µl).

**Table 1:**

| Sample volume of CHO cells (in ml) (X) | Volume of added lysis buffer (in ml) (Y) | Total final volume (in ml) |
|---|---|---|
| 5.00 | 0.71 | 5.71 |
| 6.00 | 0.86 | 6.86 |
| 7.00 | 1.00 | 8.00 |
| 8.00 | 1.14 | 9.14 |
| 9.00 | 1.29 | 10.29 |
| 10.00 | 1.43 | 11.43 |
| 11.00 | 1.57 | 12.57 |
| 12.00 | 1.71 | 13.71 |
| 13.00 | 1.86 | 14.86 |
| 14.00 | 2.00 | 16.00 |
| 15.00 | 2.14 | 17.14 |
| 16.00 | 2.29 | 18.29 |
| 17.00 | 2.43 | 19.43 |
| 18.00 | 2.57 | 20.57 |
| 19.00 | 2.71 | 21.71 |
| 20.00 | 2.86 | 22.86 |
| 21.00 | 3.00 | 24.00 |
| 22.00 | 3.14 | 25.14 |
| 23.00 | 3.29 | 26.29 |
| 24.00 | 3.43 | 27.43 |
| 25.00 | 3.57 | 28.57 |
| 26.00 | 3.71 | 29.71 |
| 27.00 | 3.86 | 30.86 |
| 28.00 | 4.00 | 32.00 |
| 29.00 | 4.14 | 33.14 |
| 30.00 | 4.29 | 34.29 |
| 31.00 | 4.43 | 35.43 |
| 32.00 | 4.57 | 36.57 |
| 33.00 | 4.71 | 37.71 |
| 34.00 | 4.86 | 37.86 |
| 35.00 | 5.00 | 40.00 |
| 36.00 | 5.14 | 41.14 |
| 37.00 | 5.29 | 42.29 |
| 38.00 | 5.42 | 43.42 |
| 39.00 | 5.57 | 44.57 |
| 40.00 | 5.71 | 45.71 |
| 41.00 | 5.86 | 46.86 |
| 42.00 | 6.00 | 48.00 |
| 43.00 | 6.14 | 49.14 |

### Example 2:

The following example illustrates the sensitivity of the method with regard to very low microorganism contamination. Several microorganisms (see Table 2) are spiked in highly concentrated fresh CHO cell samples (see Table 2). For isolating the microorganisms and their DNA the protocol of Example 1 is followed. Subsequently, the microorganisms are detected by using specific real-time PCR (25µl, performed with SYBR® Green Mastermix QuantiTect (Qiagen).

The results are given in Table 2 below:

**Table 2:**

| Species | CFU | Toltal CHO celles in sample (fresh cells) | Ct + sd (Cycle threshold) |
|---|---|---|---|
| *Enterococcus faecalis* | 71 | 2*10⁸ | 30.1 ± 0.35 |
| *Salmonella enterica* | 55 | 2.52*10⁸ | 32.07 ± 0.58 |
| *Staphylococcus aureus* | 200 | 2*10⁸ | 32.98 ± 0.95 |
| *Pseudomonas aeruginosa* | 75 | 2.58*10⁸ | 31.9 ± 0.12 |
| *Zygosaccharomyces bailii* | 93 | 3*10⁸ | 27.09 ± 1.87 |
| *Candida albicans* | 163 | 3*10⁸ | 24.56 ± 0.03 |

The results show that the method according to the present invention is able to detect bacteria, yeast and fungi down to 55 cfus in the presence of 2-3*10⁸ fresh CHO cells.

### Example 3:

The following example illustrates the sensitivity of the method with regard to very low microorganism contamination for samples of frozen CHO cells. Several microorganisms (see Table 3) are spiked in highly concentrated frozen CHO cell samples (see Table 3). For isolating the microorganisms and their DNA the protocol of Example 1 is followed. Subsequently, the microorganisms are detected by using a universal yeast and molds real-time PCR (25µl, TaqMan® PCR (Biotecon)).

The results are given in Table 3 below:

**Table 3:**

| Species | CFU | Total CHO cells in sample (frozen cells) | Ct + sd (Cycle threshold) |
|---|---|---|---|
| *Candida albicans* | 50 | | 33.53 ± -1.11 |
| *Saccharomyces cerevisae* | 38 | | 31.22 ± 0.72 |
| *Zygosaccharomyces bailii* | 38 | 3*1 0⁸ | 33.29 ± 0.35 |
| *Aspergillus brasiliensis* | 28 | | 37.58 ± 1.61 |
| *Penicillum chrysogenum* | 58 | | 35.75 ± 0.62 |

The results show that the method according to the present invention is able to detect yeast and fungi down to 28 cfus in the presence of 3*10⁸ frozen CHO cells.

## Claims

1. Method for isolating microorganisms from a sample comprising mammalian cells, comprising
a) contacting the sample with a lysis buffer comprising 2.5 to 175 U Benzonase®Nuclease, 0.05 to 0.5 % by weight SDS, 0.5 M to 4 M urea, 0.5 mM to 10 mM MgCl₂ and PBS 1X- PBS X1/8,
b) obtaining the microorganisms by removing the mammalian cells, wherein the sample is a bioreactor sample, wherein the quantity of the mammalian cells in the sample is at least 10⁶ cells.

2. Method for isolating and detecting microorganisms from a sample comprising mammalian cells, comprising
a) contacting the sample with a lysis buffer comprising 2.5 to 175 U Benzonase®Nuclease, 0.05 to 0.5 % by weight SDS, 0.5 M to 4 M urea, 0.5 mM to 10 mM MgCl₂ and PBS 1X- PBS X1/8,
b) obtaining the microorganisms by removing the mammalian cells,
c) performing a universal lysis on the microorganisms,
d) performing a specific or universal detection method for detecting the microorganisms,
wherein the sample is a bioreactor sample wherein the quantity of the mammalian cells in the sample is at least 10⁶ cells.

3. Method according to claim 1 or 2, **characterized in that** the volume of the sample is at least 5 ml.

4. Method according to one or more of claims 1 to 3, **characterized in that** the mammalian cells are selected from the group consisiting of cells growing in suspension culture, cells growing on microcarriers and/ or hybridoma cell lines.

5. Method according to one or more of claims 1 to 4, **characterized in that** the mammalian cells are selected from the group of CRL-8018 cells, CF-10H5 cells, CHO cells, NSO cells, Sp2/0 cells, HT-1080 cells, MDCK cells, HeLa cells or Vero cells.

6. Method according to one or more of claims 1 to 5, **characterized in that** the microorganisms are bacteria, yeast, fungi and/or combinations thereof.

7. Method according to one or more of claims 1 to 6, **characterized in that** in step b) centrifugation is employed.

8. Method according to one or more of claims 2 to 7, **characterized in that** in step c) sonication is employed.

9. Method according to one or more of claims 2 to 8, **characterized in that** in step d) PCR, preferably real-time PCR, is employed.

10. Use of a lysis buffer comprising the following ingredients:
- 0.05 to 0.5 % by weight SDS
- 0.5 M to 4 M urea
- 0.5 mM to 10 mM MgCl₂
- 2.5 to 175 U Benzonase®Nuclease
- PBS 1X - PBS X1/8,
in a method according to one or more of claims 1 to 9.

## Patentansprüche

1. Verfahren zum Isolieren von Mikroorganismen aus einer Probe, die Säugetierzellen umfasst, umfassend
a) Inkontaktbringen der Probe mit einem Lysepuffer, der 2,5 bis 175 U Benzonase®Nuclease, 0,05 bis 0,5 Gew.-% SDS, 0,5 M bis 4 M Harnstoff, 0,5 mM bis 10 mM MgCl₂ und PBS 1X - PBS X1/8 umfasst,
b) Erhalten der Mikroorganismen durch Entfernen der Säugetierzellen, wobei dieProbe eine Bioreaktorprobe ist, wobei die Menge der Säugetierzellen in der Probe mindestens 10⁶ Zellen beträgt.

2. Verfahren zum Isolieren und Nachweisen von Mikroorganismen aus einer Probe, die Säugetierzellen umfasst, umfassend
a) Inkontaktbringen der Probe mit einem Lysepuffer, der 2,5 bis 175 U Benzonase®Nuclease, 0,05 bis 0,5 Gew.-% SDS, 0,5 M bis 4 M Harnstoff, 0,5 mM bis 10 mM MgCl₂, und PBS 1X - PBS X1/8 umfasst,
b) Erhalten der Mikroorganismen durch Entfernen der Säugetierzellen,
c) Durchführen einer universellen Lyse an den Mikroorganismen,
d) Durchführung eines spezifischen oder universellen Nachweisverfahrens zum Nachweis der Mikroorganismen,
wobei die Probe eine Bioreaktorprobe ist, wobei die Menge der Säugetierzellen in der Probe mindestens 10⁶ Zellen beträgt.

3. Verfahren nach Anspruch 1 oder 2, **dadurch gekennzeichnet, dass** das Volumen der Probe mindestens 5 ml beträgt.

4. Verfahren nach einem oder mehreren der Ansprüche 1 bis 3, **dadurch gekennzeichnet, dass** die Säugetierzellen ausgewählt sind aus der Gruppe bestehend aus Zellen, die in Suspensionskultur wachsen, Zellen, die auf Mikroträgern wachsen und/oder Hybridom-Zellinien.

5. Verfahren nach einem oder mehreren der Ansprüche 1 bis 4, **dadurch gekennzeichnet, dass** die Säugetierzellen ausgewählt sind aus der Gruppe der CRL-8018-Zellen, CF-10H5-Zellen, CHO-Zellen, NSO-Zellen, Sp2/0-Zellen, HT-1080-Zellen, MDCK-Zellen, HeLa-Zellen oder Vero-Zellen.

6. Verfahren nach einem oder mehreren der Ansprüche 1 bis 5, **dadurch gekennzeichnet, dass** die Mikroorganismen Bakterien, Hefe, Pilze und/oder Kombinationen davon sind.

7. Verfahren nach einem oder mehreren der Ansprüche 1 bis 6, **dadurch gekennzeichnet, dass** in Schritt b) Zentrifugation verwendet wird.

8. Verfahren nach einem oder mehreren der Ansprüche 2 bis 7, **dadurch gekennzeichnet, dass** in Schritt c) Ultraschallbehandlung verwendet wird.

9. Verfahren nach einem oder mehreren der Ansprüche 2 bis 8, **dadurch gekennzeichnet, dass** in Schritt d) PCR, vorzugsweise Echtzeit-PCR, verwendet wird.

10. Verwendung eines Lysepuffers, der die folgenden Bestandteile umfasst:
- 0,05 bis 0,5 Gew.-% SDS
- 0,5 M bis 4 M Harnstoff
- 0,5 mM bis 10 mM MgCl₂
- 2,5 bis 175 U Benzonase®Nuclease
- PBS 1X - PBS X1/8,
in einem Verfahren nach einem oder mehreren der Ansprüche 1 bis 9.

## Revendications

1. Méthode d'isolement de microorganismes à partir d'un échantillon comprenant des cellules mammaliennes, comprenant
a) la mise en contact de l'échantillon avec un tampon de lyse comprenant de 2,5 à 175 U de Benzonase®Nuclease, de 0,05 à 0,5% en poids de SDS, de 0,5 M à 4 M d'urée, de 0,5 mM à 10 mM de MgCl₂ et du PBS 1X - PBS X1/8,
b) l'obtention des microorganismes par l'élimination des cellules mammaliennes,
où l'échantillon est un échantillon de bioréacteur, où la quantité des cellules mammaliennes dans l'échantillon est d'au moins 10⁶ cellules.

2. Méthode d'isolement et de détection de microorganismes à partir d'un échantillon comprenant des cellules mammaliennes, comprenant
a) la mise en contact de l'échantillon avec un tampon de lyse comprenant de 2,5 à 175 U de Benzonase®Nuclease, de 0,05 à 0,5% en poids de SDS, de 0,5 M à 4 M d'urée, de 0,5 mM à 10 mM de MgCl₂ et du PBS 1X - PBS X1/8,
b) l'obtention des microorganismes par l'élimination des cellules mammaliennes,
c) la mise en oeuvre d'une lyse universelle sur les microorganismes,
d) la mise en oeuvre d'une méthode de détection spécifique ou universelle afin de détecter les microorganismes,
où l'échantillon est un échantillon de bioréacteur, où la quantité des cellules mammaliennes dans l'échantillon est d'au moins 10⁶ cellules.

3. Méthode selon la revendication 1 ou 2, **caractérisée en ce que** le volume de l'échantillon est d'au moins 5 ml.

4. Méthode selon l'une ou plusieurs parmi les revendications 1 à 3, **caractérisée en ce que** les cellules mammaliennes sont choisies dans le groupe constitué par des cellules se développant dans une culture en suspension, des cellules se développant sur des micro-supports et/ou des lignées cellulaires d'hybridome.

5. Méthode selon l'une ou plusieurs parmi les revendications 1 à 4, **caractérisée en ce que** les cellules mammaliennes sont choisies dans le groupe constitué par les cellules CRL-8018, les cellules CF-10H5, les cellules CHO, les cellules NSO, les cellules Sp2/0, les cellules HT-1080, les cellules MDCK, les cellules HeLa ou les cellules Vero.

6. Méthode selon l'une ou plusieurs parmi les revendications 1 à 5, **caractérisée en ce que** les microorganismes sont des bactéries, de la levure, des champignons et/ou des combinaisons de ceux-ci.

7. Méthode selon l'une ou plusieurs parmi les revendications 1 à 6, **caractérisée en ce que**, dans l'étape b), une centrifugation est employée.

8. Méthode selon l'une ou plusieurs parmi les revendications 2 à 7, **caractérisée en ce que**, dans l'étape c), un traitement aux ultrasons est employé.

9. Méthode selon l'une ou plusieurs parmi les revendications 2 à 8, **caractérisée en ce que**, dans l'étape d), une PCR, préférablement une PCR en temps réel, est employée.

10. Utilisation d'un tampon de lyse comprenant les ingrédients suivants :
- de 0,05 à 0,5% en poids de SDS
- de 0,5 M à 4 M d'urée
- de 0,5 mM à 10 mM de MgCl₂
- de 2,5 à 175 U de Benzonase®Nuclease
- du PBS 1X- PBS X1/8.
dans une méthode selon l'une ou plusieurs parmi les revendications 1 à 9.
